(19) Europäisches Patentamt / European Patent Office / Office européen des brevets

(11) **EP 3 896 168 A1**

(12) **EUROPEAN PATENT APPLICATION**
published in accordance with Art. 153(4) EPC

(43) Date of publication:
**20.10.2021 Bulletin 2021/42**

(51) Int Cl.:
***C12P 21/08*** *(2006.01)*   ***C12N 5/10*** *(2006.01)*
***C12N 15/85*** *(2006.01)*

(21) Application number: **19896221.9**

(22) Date of filing: **10.12.2019**

(86) International application number:
**PCT/JP2019/048216**

(87) International publication number:
**WO 2020/122049 (18.06.2020 Gazette 2020/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.12.2018 JP 2018231592**

(71) Applicant: **FUJIFILM Corporation**
**Tokyo 106-8620 (JP)**

(72) Inventors:
• **WATANABE Yuya**
  **Ashigarakami-gun, Kanagawa 258-8577 (JP)**
• **MATSUURA Tatsuya**
  **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **ANIMAL CELLS, METHOD FOR PRODUCING ANIMAL CELLS, AND METHOD FOR PRODUCING TARGET PROTEIN**

(57) Provided are an animal cell capable of producing a target protein with high productivity, a method for producing the animal cell, and a method for producing a target protein using the animal cell. According to an aspect of the present invention, there are provided an animal cell that has a gene encoding a target protein and a foreign gene encoding SNAT2 and linked to a promoter and overexpresses SNAT2; a method for producing the animal cell; and a method for producing a target protein using the animal cell.

SEQUENCE LISTING
<110> FUJIFILM Corporation
<120> Animal cell, method for producing animal cell and method for producing protein of interest
<130>\201@18F11345W1
<160> 4
<170> PatentIn version 3.5
<210> 1
<211> 1518
<212> DNA
<213> human
<220>
<221> CDS
<222> (1)..(1518)
<400> 1

```
atg aag aag gcc gaa atg gga cga ttc agt att tcc ccg gat gaa gac      48
Met Lys Lys Ala Glu Met Gly Arg Phe Ser Ile Ser Pro Asp Glu Asp
1               5                   10                  15
agc agc agc tac agt tcc aac agc gac ttc aac tac tcc tac ccc acc      96
Ser Ser Ser Tyr Ser Ser Asn Ser Asp Phe Asn Tyr Ser Tyr Pro Thr
                20                  25                  30
aag caa gct gct ctg aaa agc cat tat gca gat gta gat cct gaa aac     144
Lys Gln Ala Ala Leu Lys Ser His Tyr Ala Asp Val Asp Pro Glu Asn
            35                  40                  45
cag aac ttt tta ctt gaa tcg aat ttg ggg aag aag aag tat gaa aca     192
Gln Asn Phe Leu Leu Glu Ser Asn Leu Gly Lys Lys Lys Tyr Glu Thr
        50                  55                  60
gaa ttt cat cca ggt act act tcc ttt gga atg tca gta ttt aat ctg     240
Glu Phe His Pro Gly Thr Thr Ser Phe Gly Met Ser Val Phe Asn Leu
65                  70                  75                  80
agc aat gcg att gtg ggc agt gga atc ctt ggg ctt tct tat gcc atg     288
Ser Asn Ala Ile Val Gly Ser Gly Ile Leu Gly Leu Ser Tyr Ala Met
                85                  90                  95
gct aat act gga att gct ctt ttt ata att ctc ttg aca ttt gtg tca     336
Ala Asn Thr Gly Ile Ala Leu Phe Ile Ile Leu Leu Thr Phe Val Ser
                100                 105                 110
ata ttt tcc ctg tat tct gtt cat ctc ctt ttg aag act gcc aat gaa     384
Ile Phe Ser Leu Tyr Ser Val His Leu Leu Leu Lys Thr Ala Asn Glu
            115                 120                 125
gga ggg tct tta tta tat gaa caa ttg gga tat aag gca ttt gga tta     432
Gly Gly Ser Leu Leu Tyr Glu Gln Leu Gly Tyr Lys Ala Phe Gly Leu
        130                 135                 140
gtt gga aag ctt gca gca tct gga tca att aca atg cag aac att gga     480
Val Gly Lys Leu Ala Ala Ser Gly Ser Ile Thr Met Gln Asn Ile Gly
145                 150                 155                 160
gct atg tca agc tac ctc ttc ata gtg aaa tat gag ttg cct ttg gtg     528
Ala Met Ser Ser Tyr Leu Phe Ile Val Lys Tyr Glu Leu Pro Leu Val
                165                 170                 175
atc cag gca tta acg aac att gaa gat aaa act gga ttg tgg tat ctg     576
Ile Gln Ala Leu Thr Asn Ile Glu Asp Lys Thr Gly Leu Trp Tyr Leu
            180                 185                 190
aac ggg aac tat ttg gtt ctg ttg gtg tca ttg gtg gtc att ctt cct     624
Asn Gly Asn Tyr Leu Val Leu Leu Val Ser Leu Val Val Ile Leu Pro
        195                 200                 205
ttg tcg ctg ttt aga aat tta gga tat ttg gga tat acc agt ggc ctt     672
Leu Ser Leu Phe Arg Asn Leu Gly Tyr Leu Gly Tyr Thr Ser Gly Leu
        210                 215                 220
tcc ttg ttg tgt atg gtg ttc ttt ctg att gtg gtc att tgc aag aaa     720
Ser Leu Leu Cys Met Val Phe Phe Leu Ile Val Val Ile Cys Lys Lys
225                 230                 235                 240
ttt cag gtt ccg tgt cct gtg gaa gct gct ttg ata att aac gaa aca     768
Phe Gln Val Pro Cys Pro Val Glu Ala Ala Leu Ile Ile Asn Glu Thr
                245                 250                 255
```

16

```
ata aac acc acc tta aca cag cca aca gct ctt gta cct gct ttg tca        816
Ile Asn Thr Thr Leu Thr Gln Pro Thr Ala Leu Val Pro Ala Leu Ser
        260             265             270
cat aac gtg act gaa aat gac tct tgc aga cct cac tat ttt att ttc        864
His Asn Val Thr Glu Asn Asp Ser Cys Arg Pro His Tyr Phe Ile Phe
        275             280             285
aac tca cag act gtc tat gct gtg cca att ctg atc ttt tca ttt gtc        912
Asn Ser Gln Thr Val Tyr Ala Val Pro Ile Leu Ile Phe Ser Phe Val
        290             295             300
tgt cat cct gct gtt ctt ccc atc tat gaa gaa ctg aaa gac cgc agc        960
Cys His Pro Ala Val Leu Pro Ile Tyr Glu Glu Leu Lys Asp Arg Ser
305             310             315             320
cgt aga aga atg atg aat gtg tcc aag att tca ttt ttt gct atg ttt       1008
Arg Arg Arg Met Met Asn Val Ser Lys Ile Ser Phe Phe Ala Met Phe
        325             330             335
ctc atg tat ctg ctt gcc gcc ctc ttt gga tac cta aca ttt tac gaa       1056
Leu Met Tyr Leu Leu Ala Ala Leu Phe Gly Tyr Leu Thr Phe Tyr Glu
        340             345             350
cat gtt gag tca gaa ttg ctt cat acc tac tct tct atc ttg gga act       1104
His Val Glu Ser Glu Leu Leu His Thr Tyr Ser Ser Ile Leu Gly Thr
        355             360             365
gat att ctt ctt ctc att gtc cgt ctg gct gtg tta atg gct gtg acc       1152
Asp Ile Leu Leu Leu Ile Val Arg Leu Ala Val Leu Met Ala Val Thr
        370             375             380
ctg aca gta cca gta gtt att ttc cca atc cgg agt tct gta act cac       1200
Leu Thr Val Pro Val Val Ile Phe Pro Ile Arg Ser Ser Val Thr His
385             390             395             400
ttg ttg tgt gca tca aaa gat ttc agt tgg tgg cgt cat agt ctc att       1248
Leu Leu Cys Ala Ser Lys Asp Phe Ser Trp Trp Arg His Ser Leu Ile
                405             410             415
aca gtg tct atc ttg gca ttt acc aat tta ctt gtc atc ttt gtc cca       1296
Thr Val Ser Ile Leu Ala Phe Thr Asn Leu Leu Val Ile Phe Val Pro
        420             425             430
act att agg gat atc ttt ggt ttt att ggt gca tct gca gct tct atg       1344
Thr Ile Arg Asp Ile Phe Gly Phe Ile Gly Ala Ser Ala Ala Ser Met
        435             440             445
ttg att ttt att ctt cct tct gcc ttc tat atc aag ttg gtg aag aaa       1392
Leu Ile Phe Ile Leu Pro Ser Ala Phe Tyr Ile Lys Leu Val Lys Lys
        450             455             460
gaa cct atg aaa tct gta caa aag att ggg gct ttg ttc ttc ctg tta       1440
Glu Pro Met Lys Ser Val Gln Lys Ile Gly Ala Leu Phe Phe Leu Leu
465             470             475             480
agt ggt gta ctg gtg atg acc gga agc atg gcc ttg att gtt ttg gat       1488
Ser Gly Val Leu Val Met Thr Gly Ser Met Ala Leu Ile Val Leu Asp
                485             490             495
tgg gta cac aat gca cct gga ggt ggc cat                                1518
Trp Val His Asn Ala Pro Gly Gly Gly His
                500             505
```

<210> 2
<211> 506
<212> PRT
<213> human
<400> 2

```
Met Lys Lys Ala Glu Met Gly Arg Phe Ser Ile Ser Pro Asp Glu Asp
1               5                   10                  15
Ser Ser Ser Tyr Ser Ser Asn Ser Asp Phe Asn Tyr Ser Tyr Pro Thr
                20                  25                  30
Lys Gln Ala Ala Leu Lys Ser His Tyr Ala Asp Val Asp Pro Glu Asn
        35                  40                  45
Gln Asn Phe Leu Leu Glu Ser Asn Leu Gly Lys Lys Lys Tyr Glu Thr
        50                  55                  60
Glu Phe His Pro Gly Thr Thr Ser Phe Gly Met Ser Val Phe Asn Leu
65                  70                  75                  80
```

```
Ser Asn Ala Ile Val Gly Ser Gly Ile Leu Gly Leu Ser Tyr Ala Met
                85                      90                  95
Ala Asn Thr Gly Ile Ala Leu Phe Ile Leu Leu Thr Phe Val Ser
            100                 105                 110
Ile Phe Ser Leu Tyr Ser Val His Leu Leu Leu Lys Thr Ala Asn Glu
            115                 120                 125
Gly Gly Ser Leu Leu Tyr Glu Gln Leu Gly Tyr Lys Ala Phe Gly Leu
    130                 135                 140
Val Gly Lys Leu Ala Ala Ser Gly Ser Ile Thr Met Gln Asn Ile Gly
145                 150                 155                 160
Ala Met Ser Ser Tyr Leu Phe Ile Val Lys Tyr Glu Leu Pro Leu Val
            165                 170                 175
Ile Gln Ala Leu Thr Asn Ile Glu Asp Lys Thr Gly Leu Trp Tyr Leu
            180                 185                 190
Asn Gly Asn Tyr Leu Val Leu Leu Val Ser Leu Val Val Ile Leu Pro
        195                 200                 205
Leu Ser Leu Phe Arg Asn Leu Gly Tyr Leu Gly Tyr Thr Ser Gly Leu
    210                 215                 220
Ser Leu Leu Cys Met Val Phe Phe Leu Ile Val Val Ile Cys Lys Lys
225                 230                 235                 240
Phe Gln Val Pro Cys Pro Val Glu Ala Ala Leu Ile Ile Asn Glu Thr
            245                 250                 255
Ile Asn Thr Thr Leu Thr Gln Pro Thr Ala Leu Val Pro Ala Leu Ser
            260                 265                 270
His Asn Val Thr Glu Asn Asp Ser Cys Arg Pro His Tyr Phe Ile Phe
        275                 280                 285
Asn Ser Gln Thr Val Tyr Ala Val Pro Ile Leu Ile Phe Ser Phe Val
    290                 295                 300
Cys His Pro Ala Val Leu Pro Ile Tyr Glu Glu Leu Lys Asp Arg Ser
305                 310                 315                 320
Arg Arg Arg Met Met Asn Val Ser Lys Ile Ser Phe Phe Ala Met Phe
            325                 330                 335
Leu Met Tyr Leu Leu Ala Ala Leu Phe Gly Tyr Leu Thr Phe Tyr Glu
            340                 345                 350
His Val Glu Ser Glu Leu Leu His Thr Tyr Ser Ser Ile Leu Gly Thr
        355                 360                 365
Asp Ile Leu Leu Leu Ile Val Arg Leu Ala Val Leu Met Ala Val Thr
    370                 375                 380
Leu Thr Val Pro Val Val Ile Phe Pro Ile Arg Ser Ser Val Thr His
385                 390                 395                 400
Leu Leu Cys Ala Ser Lys Asp Phe Ser Trp Trp Arg His Ser Leu Ile
            405                 410                 415
Thr Val Ser Ile Leu Ala Phe Thr Asn Leu Leu Val Ile Phe Val Pro
            420                 425                 430
Thr Ile Arg Asp Ile Phe Gly Phe Ile Gly Ala Ser Ala Ala Ser Met
            435                 440                 445
Leu Ile Phe Ile Leu Pro Ser Ala Phe Tyr Ile Lys Leu Val Lys Lys
    450                 455                 460
Glu Pro Met Lys Ser Val Gln Lys Ile Gly Ala Leu Phe Phe Leu Leu
465                 470                 475                 480
Ser Gly Val Leu Val Met Thr Gly Ser Met Ala Leu Ile Val Leu Asp
            485                 490                 495
Trp Val His Asn Ala Pro Gly Gly Gly His
            500                 505

<210>   3
<211>   25
<212>   DNA
<213>   Artificial Sequence
<220>
<223>   Description of Artificial Sequence: primer
<400>   3
ccgcggtcat agctgtttcc tgaac                                        25
<210>   4
```

```
<211>   43
<212>   DNA
<213>   Artificial Sequence
<220>
<223>   Description of Artificial Sequence: primer
<400>   4
cagctatgac cgcggttaat ggccacctcc aggtgcattg tgt                     43
```

**Description**

Field of the Invention

The present invention relates to animal cells that express a target protein. An aspect of the present invention relates to a method for producing the above-mentioned animal cells, and a method for producing a target protein using the above-mentioned animal cells.

Description of the Related Art

In producing biopharmaceuticals such as antibodies, fed-batch culture, which improves a state of cells by adding nutrients to a culture solution, is often used to improve the productivity of antibodies. In addition, in producing biopharmaceuticals such as antibodies, a perfusion culture method, in which a culture solution is continuously filtered and discharged while a fresh medium containing nutrients is continuously fed to a culture tank, is often used to improve the productivity of antibodies.

Patent Document 1 discloses a method for producing polypeptide, including strongly expressing alanine aminotransferase, culturing cells in which DNA encoding a desired polypeptide is introduced, and producing the desired polypeptide, as a method capable of producing protein in a high production amount.

Prior Art Documents

Patent Documents

Patent Document 1: WO2009/020144

**SUMMARY OF THE INVENTION**

In fed-batch culture, it is possible to perform cell culture for a long period and at a higher cell density compared to a case where nutrients are not additionally added, but there is a problem in that cell viability is degraded in the latter half of the culture due to accumulation of waste products secreted from cells, and thus products cannot be increased by extended culture period.

In addition, there is a problem in that in order to culture cells at high density in the perfusion culture method, the cost of the culture is increased since harvesting and supplying of a medium in an amount of 1 to 3 times the culture volume per day is required to supply nutrients and discharge waste products out of the system.

An object of an aspect of the present invention is to provide an animal cell capable of producing a target protein with high productivity. Another object of an aspect of the present invention is to provide a method for producing the animal cell and a method for producing a target protein using the animal cell.

The present inventors have conducted intensive studies to achieve the above-mentioned objects, and as a result, the present inventors found that the production amount of protein is affected by forcibly expressing a sodium-dependent neutral amino acid transporter-2 (SNAT2) gene (gene name is SLC38A2) in CHO cells, and thus it is possible to achieve improvement of antibody productivity (Qp) and improvement of antibody production amount. Aspects of the present invention was completed based on the above findings.

That is, according to an aspect of the present invention, the following inventions are provided.

<1> An animal cell that has a gene encoding a target protein and a foreign gene encoding SNAT2 and linked to a promoter, and overexpresses the SNAT2.

<2> The animal cell according to <1>, in which the overexpression is constant.

<3> The animal cell according to <1> or <2>, in which the foreign gene encoding SNAT2 has a base sequence having 90% or more sequence identity with a base sequence of SEQ ID NO: 1.

<4> The animal cell according to any one of <1> to <3>, in which the foreign gene encoding SNAT2 includes a base sequence of SEQ ID NO: 1.

<5> The animal cell according to any one of <1> to <4>, in which the animal cell is a CHO cell.

<6> A method for producing the animal cell according to any one of <1> to <5>, the method including a step of introducing a gene encoding a target protein and a foreign gene encoding SNAT2 and linked to a promoter, into an animal cell.

<7> The method according to <6>, in which the step of introducing a foreign gene encoding SNAT2 and linked to a promoter is performed by electroporation.

<8> A method for producing a target protein, including culturing the animal cell according to any one of <1> to <5>.

<9> The method according to <8>, in which the culture is fed-batch culture.

<10> The method according to <9>, in which a seeded cell density of a cell culture is 0.2 x $10^6$ cells/mL to 5 x $10^6$ cells/mL.

<11> The method according to <10>, in which a viable cell rate during a culture period is 60% or more over the entire period.

<12> The method according to <8>, in which the culture is perfusion culture.

<13> The method according to <12>, in which the seeded cell density of the cell culture is 0.2 $\times 10^6$ cells/mL to 1 x $10^7$ cells/mL.

<14> The method according to <13>, in which a viable cell rate during a culture period is 90% or more over the entire period.

According to the animal cell of an aspect of the present invention, it is possible to produce a target protein with high productivity.

## BRIEF DESCRIPTION OF THE DRAWINGS

Fig. 1 illustrates a result of measuring an antibody concentration in a culture supernatant of cells after gene introduction.

Fig. 2 illustrates a result of measuring antibody productivity (Qp) per cell in the culture supernatant of cells after gene introduction.

Fig. 3 illustrates a result of measuring a cell size after gene introduction.

Fig. 4 illustrates a result of measuring the cumulative number of viable cells during a culture period.

## DESCRIPTION OF THE PREFERRED EMBODIMENTS

Hereinafter, embodiments for carrying out an aspect of the present invention will be described in detail.

In the present specification, a numerical value range indicated by using "to" means a range including numerical values described before and after the "to" as a minimum value and a maximum value, respectively.

[Animal cells]

An animal cell of an aspect of the present invention is an animal cell that includes a gene encoding a target protein and a foreign gene encoding SNAT2 and linked to a promoter, and overexpresses the SNAT2.

The expression of the SNAT2 gene activates the mTORC1/S6K pathway in a mouse liver (Nature Communications 6, Article number: 7940 (2015)), and this thus suggests a relationship between the expression of the SNAT2 gene and the expression of the mTOR gene. It has been reported that in rat cells, cell proliferation is suppressed by activation of mTOR and cell size is increased (Genes Dev. 2002 Jun 15; 16 (12): 1472-1487). In addition, it has also been shown that the larger the cell size, the greater the production amount of a protein product (Cytotechnology 34: 59-70, 2000). However, the relationship between SNAT2 gene expression and the production amount of recombinant protein is unclear.

In WO2009/020144, a decrease in survival rate was moderated due to the effect of introducing alanine aminotransferase, but since the period that survival rate of cells is 60% or less is long, there is concern about the influence on antibody quality. In addition, since the antibody productivity (Qp) per cell is lowered by the introduction of the taurine transporter, there is a problem that even if the cell density is increased, it does not lead to the production amount. On the other hand, in an aspect of the present invention, antibody productivity (Qp) per cell is improved by enhancing the expression of the SNAT2 gene involved in protein biosynthesis.

<Target protein>

In an aspect of the present invention, the kind of the target protein is not particularly limited, and examples thereof include a recombinant polypeptide chain, a recombinant secreted polypeptide chain, an antigen-binding protein, a human antibody, a humanized antibody, a chimeric antibody, a mouse antibody, a bispecific antibody, and an Fc fusion protein, a fragmented immunoglobulin, and a single-chain antibody (scFv). The target protein is preferably a human antibody, a humanized antibody, a chimeric antibody, or a mouse antibody. Examples of the fragmented immunoglobulin include Fab, F(ab')$_2$, Fv, and the like. The class of the antibody is also not particularly limited, and may be any one class of IgG such as IgG1, IgG2, IgG3, and IgG4, IgA, IgD, IgE, or IgM, but in a case of being used as a medicine, IgG and IgM are preferable.

Human antibodies include all antibodies having one or a plurality of variable and constant domains introduced from human immunoglobulin sequences. In one embodiment, all of the variable and constant domains are introduced from human immunoglobulin sequences (complete human antibodies).

The humanized antibody has a sequence different from a sequence of an antibody introduced from a non-human species by substitution, deletion, and/or addition of one or a plurality of amino acids, so that there is a low possibility for the humanized antibody to induce an immune response, and/or so that induction of a severe immune response is reduced, compared to the non-human species antibody, in a case of being administered to a human subject. In one embodiment, specific amino acids in a framework and constant domains of heavy chains and/or light chains of a non-human species antibody are mutated to produce a humanized antibody. In another embodiment, the constant domains of human antibodies are fused to variable domains of a non-human antibodies.

The chimeric antibody is an antibody in which variable domains and constant domains having different origins from each other are linked. For example, an antibody consisting of variable domains of heavy chains and light chains of a mouse antibody and the constant domains of heavy chains and light chains of a human antibody is a mouse/human heterologous chimeric antibody. It is possible to prepare a recombinant vector expressing a chimeric antibody by linking a DNA encoding the variable domains of a mouse antibody and a DNA encoding the constant domains of a human antibody, and incorporating thereof into an expression vector. It is possible to obtain a chimeric antibody produced during the culture by culturing a recombinant cell transformed with the vector and expressing the incorporated DNA.

A bispecific antibody is an antibody that recognizes two different antigenic specificities and are prepared by a chemical method or cell fusion. As a method for preparing a bispecific antibody, a method of preparing a bispecific antibody by binding two immunoglobulin molecules by using a cross-linking agent such as N-succinimidyl 3-(2-pyridyldithiol) propionate or S-acetylmercaptosuccinic acid anhydride, a method of preparing a bispecific antibody by binding Fab fragments of immunoglobulin molecules with each other, and the like have been reported.

An Fc fusion protein indicates a protein having an Fc domain and includes an antibody.

Fab is a monovalent fragment having $V_L$, $V_H$, $C_L$ and $C_H1$ domains.

$F(ab')_2$ is a bivalent fragment having two Fab fragments bound by a disulfide cross-linking at a hinge domain.

The Fv fragment has a single arm $V_L$ and $V_H$ domains of an antibody.

Single-chain antibody (scFv) is an antibody in which the $V_L$ and $V_H$ domains are joined via a linker (for example, a synthetic sequence of an amino acid residue) to form a continuous protein chain, in which the linker is long enough to fold the protein chain on itself and to form a monovalent antigen binding site.

A gene encoding a target protein can be obtained by a method known to those skilled in the art. In a case where the target protein is an antibody, it is possible to use a DNA encoding the L chain and a DNA encoding the H chain of the antibody.

It is possible to prepare the DNA encoding the L chain and the DNA encoding the H chain of the antibody as follows. An mRNA is extracted from a hybridoma, a cell, a phage, a ribosome, or the like having a gene that expresses an antibody. A cDNA is prepared by a reverse transcription reaction using a reverse transcriptase from the mRNA. The L chain gene or the H chain gene is amplified by PCR using a primer and a cDNA having a base sequence complementary to the L chain gene or the H chain gene, and each gene is obtained by binding to a cloning plasmid.

It is possible to prepare the DNA encoding the L chain fragment and the DNA encoding the H chain fragment of the antibody as follows. An mRNA is extracted from a hybridoma, a cell, a phage, a ribosome, or the like having a gene that expresses an antibody. A cDNA is prepared by a reverse transcription reaction using a reverse transcriptase from the mRNA. The L chain gene fragment or the H chain gene fragment is amplified by PCR using a primer having a base sequence complementary to the L chain gene fragment or the H chain gene fragment and a cDNA, and each gene fragment is obtained by binding to a cloning plasmid.

<SNAT2 and SNAT2 genes>

In an aspect of the present invention, the origin of SNAT2 is not particularly limited, and a foreign gene encoding SNAT2 derived from a mammal such as human, monkey, mouse, rat, or hamster can be used. In an aspect of the present invention, the foreign gene refers to a gene introduced into an animal cell from outside. In addition, even in a case where an endogenous gene is amplified to introduce a gene, it is regarded as a foreign gene.

The base sequence and the amino acid sequence of human SNAT2 are shown in SEQ ID NOs: 1 and 2 in the sequence list.

Base sequence of human SNAT2 gene (SEQ ID NO: 1)

ATGAAGAAGGCCGAAATGGGACGATTCAGTATTTCCCCGGATGAAGACAG CAGCAGCTACAGTTCCAACA

GCGACTTCAACTACTCCTACCCCACCAAGCAAGCTGCTCTGAAAAGCCATT ATGCAGATGTAGATCCTGA

AAACCAGAACTTTTTACTTGAATCGAATTTGGGGAAGAAGAAGTATGAAA CAGAATTTCATCCAGGTACT

ACTTCCTTTGGAATGTCAGTATTTAATCTGAGCAATGCGATTGTGGGCAGTG GAATCCTTGGGCTTTCTT

ATGCCATGGCTAATACTGGAATTGCTCTTTTTATAATTCTCTTGACATTTGTG TCAATATTTTCCCTGTA

TTCTGTTCATCTCCTTTTGAAGACTGCCAATGAAGGAGGGTCTTTATTATAT GAACAATTGGGATATAAG

GCATTTGGATTAGTTGGAAAGCTTGCAGCATCTGGATCAATTACAATGCAG

AACATTGGAGCTATGTCAA

GCTACCTCTTCATAGTGAAATATGAGTTGCCTTTGGTGATCCAGGCATTAAC GAACATTGAAGATAAAAC

TGGATTGTGGTATCTGAACGGGAACTATTTGGTTCTGTTGGTGTCATTGGTG GTCATTCTTCCTTTGTCG

CTGTTTAGAAATTTAGGATATTTGGGATATACCAGTGGCCTTTCCTTGTTGTG TATGGTGTTCTTTCTGA

TTGTGGTCATTTGCAAGAAATTTCAGGTTCCGTGTCCTGTGGAAGCTGCTT TGATAATTAACGAAACAAT

AAACACCACCTTAACACAGCCAACAGCTCTTGTACCTGCTTTGTCACATAA CGTGACTGAAAATGACTCT

TGCAGACCTCACTATTTTATTTTCAACTCACAGACTGTCTATGCTGTGCCAA TTCTGATCTTTTCATTTG

TCTGTCATCCTGCTGTTCTTCCCATCTATGAAGAACTGAAAGACCGCAGCC GTAGAAGAATGATGAATGT

GTCCAAGATTTCATTTTTTGCTATGTTTCTCATGTATCTGCTTGCCGCCCTCT TTGGATACCTAACATTT

TACGAACATGTTGAGTCAGAATTGCTTCATACCTACTCTTCTATCTTGGGAA CTGATATTCTTCTTCTCA

TTGTCCGTCTGGCTGTGTTAATGGCTGTGACCCTGACAGTACCAGTAGTTAT TTTCCCAATCCGGAGTTC

TGTAACTCACTTGTTGTGTGCATCAAAAGATTTCAGTTGGTGGCGTCATAGT CTCATTACAGTGTCTATC

TTGGCATTTACCAATTTACTTGTCATCTTTGTCCCAACTATTAGGGATATCTT TGGTTTTATTGGTGCAT

CTGCAGCTTCTATGTTGATTTTTATTCTTCCTTCTGCCTTCTATATCAAGTTG GTGAAGAAAGAACCTAT

GAAATCTGTACAAAGATTGGGGCTTTGTTCTTCCTGTTAAGTGGTGTACT GGTGATGACCGGAAGCATG

GCCTTGATTGTTTTGGATTGGGTACACAATGCACCTGGAGGTGGCCAT

Amino acid sequence of human SNAT2 gene (SEQ ID NO: 2)

MKKAEMGRFSISPDEDSSSYSSNSDFNYSYPTKQAALKSHYADVDPENQNFL LESNLGKKKYETEFHPGT

TSFGMSVFNLSNAIVGSGILGLSYAMANTGIALFIILLTFVSIFSLYSVHLLLKTA NEGGSLLYEQLGYK

AFGLVGKLAASGSITMQNIGAMSSYLFIVKYELPLVIQALTNIEDKTGLWYLN GNYLVLLVSLVVILPLS

LFRNLGYLGYTSGLSLLCMVFFLIVVICKKFQVPCPVEAALIINETINTTLTQPT ALVPALSHNVTENDS

CRPHYFIFNSQTVYAVPILIFSFVCHPAVLPIYEELKDRSRRRMMNVSKISFFAM FLMYLLAALFGYLTF

YEHVESELLHTYSSILGTDILLLIVRLAVLMAVTLTVPVVIFPIRSSVTHLLCAS KDFSWWRHSLITVSI

LAFTNLLVIFVPTIRDIFGFIGASAASMLIFILPSAFYIKLVKKEPMKSVQKIGAL FFLLSGVLVMTGSM

ALIVLDWVHNAPGGGH

As a foreign gene encoding SNAT2,

(1) a gene encoding a protein consisting of an amino acid sequence of SEQ ID NO: 2;
(2) a gene encoding a protein consisting of an amino acid sequence in which one or several amino acids are deleted, substituted, or added in the amino acid sequence of SEQ ID NO: 2, and having SNAT2 function; or
(3) a gene encoding a protein consisting of an amino acid sequence having 85% or more (more preferably 90% or more, particularly preferably 95% or more, most preferably 98% or more) sequence identity with the amino acid sequence of SEQ ID NO: 2, and having SNAT2 function;
can be used.

The SNAT2 function means a function of activating the mTORC1/S6K pathway. In rat cells, it is known that activation of mTOR suppresses cell proliferation and increases cell size.

As a foreign gene encoding SNAT2,

(4) a gene consisting of a base sequence of SEQ ID NO: 1;
(5) a gene consisting of a base sequence in which one or several bases are deleted, substituted or added in the base sequence of SEQ ID NO: 1, and encoding a protein having SNAT2 function;
(6) a gene consisting of a base sequence that hybridizes under stringent conditions to a sequence complementary to the base sequence of SEQ ID NO: 1 and encoding a protein having SNAT2 function; or
(7) a gene having a base sequence having 90% or more (more preferably 95% or more, particularly preferably 98% or more) sequence identity with the base sequence of SEQ ID NO: 1 and encoding a protein having SNAT2 function:
can also be used.

"One or several" in "amino acid sequence in which one or several amino acids are deleted, substituted or added" means preferably 1 to 20, more preferably 1 to 10, further more preferably 1 to 5, and particularly preferably 1 to 3.

The sequence identity in an aspect of the present invention indicates a value calculated by the following formula.

$$\% \text{ Sequence identity} = [(\text{number of identical residues})/(\text{longer alignment length})] \times 100$$

The sequence identity between two amino acid sequences can be determined by any method known to those skilled in the art, and can be determined using the Basic Local Alignment Search Tool (BLAST) program (J. Mol. Biol. 215: 403-410, 1990) or the like. The "longer alignment length" of the denominator means that in a case where two alignments are compared, the longer alignment length is used as the denominator.

"One or several" in the "base sequence in which one or several bases are deleted, substituted or added" means preferably 1 to 20, more preferably 1 to 10, further more preferably 1 to 5, and particularly preferably 1 to 3.

"Under the stringent conditions" in "hybridizes under stringent conditions" means hybridizing under moderately or highly stringent conditions, which can be recognized by those skilled in the art. Examples of the moderately stringent conditions include conditions described in Sambrook et al., Molecular Cloning: A Laboratory Manual, Third Edition, Vol. 1, 7. 42-7. 45 Cold Spring Harbor Laboratory Press, 2001. Examples of the moderately stringent conditions include a hybridization conditions of a pre-wash solution of 5x SSC, 0.5% SDS, and 1.0 mmol/L EDTA (pH 8.0), approximately 50% formamide at approximately 40°C to 50°C, and 2x SSC to 6x SSC (or other similar hybridization solutions such as Stark's solution in approximately 50% formamide at approximately 42°C) in a nitrocellulose filter, and washing conditions of approximately 60°C, 0.5x SSC, and 0.1% SDS. Highly stringent conditions can also be easily determined by those skilled in the art, and examples thereof include hybridization and/or washing at a higher temperature and/or a lower salt concentration than the above-described moderately stringent conditions. For example, the above-described hybridization conditions and washing at 68°C, 0.2x SSC, and 0.1% SDS can be exemplified. Here, the composition of $1\times$ SSC is 150 mmol/L NaCl, 15 mmol/L sodium citrate, and pH 7.4. SDS is sodium dodecyl sulfate, and EDTA is ethylene diamine tetraacetic acid.

Information on the amino acid sequence and the base sequence of non-human SNAT2 is shown below. The following numbers indicate the Gene No. of National Center for Biotechnology Information (NCBI).

ID: 29642 rat (Rattus norvegicus)
ID: 67760 mouse (Mus musculus)
ID: 566537 zebrafish (Danio rerio)
ID: 338044 cattle (Bos taurus)
ID: 417807 chicken (Gallus gallus)
ID: 702253 Rhesus monkey (Macaca mulatta)
ID: 101083348 domestic cat (Felis catus)
ID: 100525644 pig (Sus scrofa)
ID: 101149564 gorilla (Gorilla gorilla)

For non-human SNAT2, as in the case of human SNAT2,

(2A) a gene encoding a protein consisting of an amino acid sequence in which one or several amino acids are deleted, substituted or added in a predetermined amino acid sequence, and having SNAT2 function;
(3A) a gene encoding a protein consisting of an amino acid sequence having 85% or more (more preferably 90% or more, particularly preferably 95% or more, and most preferably 98% or more) sequence identity with a predetermined amino acid sequence, and having SNAT2 function;
(5A) a gene encoding a protein consisting of a base sequence in which one or several bases are deleted, substituted or added in a predetermined base sequence and having SNAT2 function;
(6A) a gene encoding a protein consisting of a base sequence that hybridizes under stringent conditions to a sequence complementary to a predetermined base sequence, and having SNAT2 function; or
(7A) a gene having a base sequence having 90% or more (more preferably 95% or more, particularly preferably 98% or more) sequence identity with a predetermined base sequence and encoding a protein having SNAT2 function: may be used.

The foreign gene encoding SNAT2 is linked to the promoter.

The promoter is not particularly limited as long as it can function in animal cells of a host and make the animal cells to express SNAT2. The promoter is preferably cytomegalovirus promoter (CMV promoter), EF1α promoter (promoter of human polypeptide chain elongation factor gene), Simian Virus 40 promoter (SV40 promoter), β-actin promoter, MMLV-LTR promoter (promoter of long terminal repeat of moloney murine leukemia virus), or mouse β-globin promoter, and the CMV promoter is more preferable.

In the animal cells of an aspect of the present invention, SNAT2 is overexpressed. Overexpression means that expression of a certain gene exceeds a normal expression level in a host. By introducing a foreign gene encoding SNAT2 into a host and making the foreign gene express SNAT2 in the host, an animal cell overexpressing SNAT2 can be obtained.

The expression level of the SNAT2 in the animal cells of an aspect of the present invention is preferably 3 times or more, more preferably 3.5 times or more, further more preferably 4 times or more, more preferably 4.5 times or more, further more preferably 5 times or more, and particularly preferably 5.5 times or more that of animal cells not having a foreign gene encoding SNAT2. There may be no upper limit, but it may be 30,000 times or less, or may be 10,000 times or less.

The expression level of the SNAT2 can be examined by reverse transcription-polymerase chain reaction (RT-PCR) method and the like. Measurement of the expression level of SNAT2 is preferably carried out by reverse transcription of mRNA and real-time PCR. The expression level of the SNAT2 is preferably a relative expression level calculated by normalization. Normalization can be performed, for example, by comparative quantification using the expression level of a housekeeping gene such as β-actin or HPRT1 as an endogenous control.

<Animal cells>

The cells in an aspect of the present invention are not particularly limited as long as the cells are animal cells. Examples of the animal cells include Chinese hamster ovary (CHO) cells, BHK cells, 293 cells, myeloma cells (such as NS0 cells), PerC6 cells, SP2/0 cells, hybridoma cells, COS cells, 3T3 cells, HeLa cells, Vero cells, MDCK cells, PC12 cells, and WI38 cells. Among these, particularly CHO cells, BHK cells, 293 cells, myeloma cells (such as NS0 cells), PerC6 cells, SP2/0 cells, and hybridoma cells are preferable, and CHO cells are more preferable. The CHO cells are widely used for production of recombinant proteins such as cytokines, coagulation factors, and antibodies. It is preferable to use CHO cells deficient in dihydrofolate reductase (DHFR), and as DHFR-deficient CHO cells, for example, CHO-DG44 can be used.

[Method for producing animal cells]

According to an aspect of the present invention, there is provided a method for producing an animal cell of an aspect of the present invention, including a step of introducing a gene encoding a target protein and a foreign gene encoding SNAT2 and linked to a promoter into animal cells.

It is preferable that the gene encoding a target protein and the foreign gene encoding SNAT2 are each incorporated into a vector and introduced into a host animal cell.

As the vector that can be used to introduce a gene into a host, it is possible to use a mammalian-derived expression vector. Examples thereof include pCMV6-Entry (manufactured by OriGene Technologies, Inc.), pcDNA3 (manufactured by Invitrogen), pEGF-BOS (Nucleic Acids. Res. 1990, 18 (17), p5322), pEF, pCDM8 (manufactured by Funakoshi Co., Ltd.), INPEP4 (manufactured by Biogen-IDEC), and the like, but are not particularly limited thereto.

In addition, it is known that mRNA having polyA is stable in cells. The gene encoding a target protein and the foreign gene encoding SNAT2 may have polyA signals necessary for adding polyA to the gene, for example, mouse β-globin polyA signal, bovine growth hormone polyA signal, SV40 polyA signal, and the like.

The method for introducing a gene encoding a target protein and a foreign gene encoding SNAT2 into animal cells is not particularly limited, and the introduction can be performed by a method known to those skilled in the art. For example, it is possible to perform electroporation, lipofection, a calcium phosphate method, a DEAE dextran method, a method using cationic liposome DOTAP (manufactured by Roche diagnostics), or a method using a virus vector. Among these, electroporation is preferable.

In a case where a gene is introduced into an animal cell, the gene is introduced into only some of the cells subjected to gene introduction, depending on the kind of the expression vector used and the gene introduction method. In order to identify and select cells into which the gene has been introduced, for example, a gene encoding a selectable marker for resistance to antibiotics may be introduced into the host cell along with the target gene. Preferable selectable markers include those conferring resistance to drugs, such as G418, hygromycin, and methotrexate.

In the cells of an aspect of the present invention, the gene encoding a target protein may be expressed in a transient expression system, or may be expressed in a constant expression system, but may be preferably expressed in the constant expression system.

In the cells of an aspect of the present invention, the foreign gene encoding SNAT2 may be expressed in a transient expression system, or may be expressed in a constant expression system, but may be preferably expressed in the constant expression system.

The transient expression system is a system in which a cyclic plasmid is incorporated into cells and made to express by a calcium phosphate method, an electroporation method, a lipofection method, or the like. A gene encoding a target protein and a foreign gene encoding SNAT2 often exist outside the chromosome.

The constant expression system is a system in which a cyclic plasmid or a linear plasmid created by restriction enzyme treatment is incorporated into cells by a calcium phosphate method, an electroporation method, a lipofection method, or the like, and a part thereof is inserted into a cell genome to express a target protein. It is possible to maintain expression of the gene encoding a target protein and the foreign gene encoding SNAT2 for a long period of time. In addition, drug selection is possible by introduction of a drug resistant gene into a plasmid, and it is possible to efficiently select the cells in which the gene encoding a target protein and the foreign gene encoding SNAT2 are maintained on chromosomes.

[Method for producing target protein]

According to the present invention, there is provided a method for producing a target protein, including culturing an animal cell of an aspect of the present invention.

It is possible to produce a target protein by culturing the animal cell of an aspect of the present invention. A culture can be performed according to a known method.

As a medium used for culturing animal cells of an aspect of the present invention, a medium used for culturing normal animal cells can be used. For example, an OptiCHO (Life Technologies, 12681011) medium, a Dulbecco's modified eagle medium (DMEM), an eagle's minimum essential medium (MEM), an RPMI-1640 medium, an RPMI-1641 medium, a F-12K medium, a Ham F12 medium, an Iscove's modified method Dulbecco's medium (IMDM), a McCoy's 5A medium, a Leibovitz L-15 medium, and an EX-CELL (trademark) 300 series (JRH Biosciences), a CHO-S-SFMII (Invitrogen Corporation), a CHO-SF (Sigma-Aldrich), a CD-CHO (Invitrogen Corporation), an IS CHO-V (Irvine Scientific), a PF-ACF-CHO (Sigma-Aldrich Corporation), or the like can be used.

Serum such as fetal calf serum (FCS) may be added to the medium, and culture in a serum-free medium may be more preferable and culture in a total synthesis medium may be most preferable.

The medium may be supplemented with additional components such as amino acids, salts, sugars, vitamins, hormones, proliferation factors, buffers, antibiotics, lipids, trace elements, and hydrolysates of plant proteins.

The pH of the medium varies depending on the cells to be cultured, but is generally pH 6.0 to 8.0, preferably pH 6.8 to 7.6, and more preferably pH 7.0 to 7.4.

The culture temperature is generally 30°C to 40°C, preferably 32°C to 37°C, and more preferably 36°C to 37°C, and the culture temperature may be changed during the culture.

The culture is preferably performed in an atmosphere having a $CO_2$ concentration of 0% to 40%, and preferably 2% to 10%.

The culture time is not particularly limited, but is generally 12 hours to 90 days, preferably 24 hours to 60 days, more preferably 24 hours to 30 days.

In the culture, the medium can be replaced, aerated, and agitated depending on the necessity.

The animal cells of an aspect of the present invention can be cultured in a culture device (also referred to as a bioreactor) or other suitable containers. Examples of the culture device include a fermenter tank culture device, an air lift culture device, a culture flask culture device, a spinner flask culture device, a microcarrier culture device, a fluidized bed culture device, a hollow fiber culture device, a roller bottle culture device, a filling tank culture device, and the like.

The culture scale is generally 1 L to 20,000 L, preferably 1 L to 10,000 L, more preferably 200 L to 2,000 L, and further more preferably 500 L to 2,000 L.

As the culture, any method such as batch culture, fed-batch culture (also referred to as feeding culture), or perfusion culture may be used, but fed-batch culture or perfusion culture is preferable.

The batch culture is a discontinuous method in which cells are proliferated for a short period in a fixed-volume culture medium, and then are completely harvested. A culture product proliferated using the batch method experiences an increase in cell density until reaching a maximum cell density, and then medium components are consumed and levels of metabolic by-products (lactate, ammonia, and the like) accumulate, which causes a viable cell density to decline. Harvesting is typically performed at a time when the maximum cell density (typically, 5 to 10 $\times$ 10^6 cells/mL) is achieved. A batch process is the simplest culture method, but the viable cell density is limited by nutrient availability, and once the cells reach the maximum density, the culture is declined and the production of the target protein is reduced. Since accumulation of waste products and nutrient depletion rapidly lead to culture decline (typically approximately 3 to 7 days), it is not possible to extend the duration of production of the target protein.

The fed-batch culture is a culture method that improves the batch process by feeding a bolus or continuous medium and replenishing the consumed medium components. That is, in the fed-batch culture, the culture form is suspension culture, which provides additional components to the culture at one or more points after the start of the culture process. Examples of the additional components include nutrition supplement components for cells that are depleted during the culture process, and may include other auxiliary components (for example, cell cycle inhibiting compounds).

In the fed-batch culture, since additional nutrients are added throughout the culture period, there is a possibility that higher cell densities and higher yields of the target protein may be achieved compared to the batch culture. In the fed-batch culture, unlike the batch culture, it is possible to prepare and maintain a two-phase culture by operating the feeding schedule and the culture components such that a period (proliferation phase) of cell proliferation for achieving a desired cell density is distinguished from a period (production period) of stopped or slow cell proliferation. In this manner, there is a possibility that the fed-batch culture can achieve a higher production amount of the target protein compared to the batch culture.

In fed-batch culture, a seeded cell density of the cell culture is generally $0.2 \times 10^6$ cells/mL to $1 \times 10^7$ cells/mL, preferably $0.2 \times 10^6$ cells/mL to $5 \times 10^6$ cells/mL, more preferably $0.5 \times 10^6$ cells/mL to $2.5 \times 10^6$ cells/mL, and further more preferably $0.5 \times 10^6$ cells/mL to $1.5 \times 10^6$ cells/mL.

In the fed-batch culture, the viable cell rate during the culture period is preferably 60% to 100%, more preferably 70% to 100%, and further more preferably 75% to 100% in the entire period.

A perfusion culture is a culture method in which a fresh medium is added and a used medium is removed at the same time, and there is a possibility that the batch culture and the fed-batch culture can be further improved. The perfusion culture can be performed using an Alternating Tangential Flow Filtration (ATF) pump or a Tangential Flow Filtration (TFF) pump, for example. According to the perfusion culture, it is possible to achieve a high cell density exceeding $1 \times 10^8$ cells/mL. A typical perfusion culture starts with a batch culture start-up lasting one or two days, then, a fresh feed medium is continuously, in phases, and/or intermittently added to the culture product, and the used medium is removed at the same time. In the perfusion culture, it is possible to remove a used medium while maintaining a cell density using a method such as sedimentation, centrifugation, or filtration. The perfusion culture can be performed using an ATF pump or a TFF pump, for example.

The advantage of the perfusion culture is that the culture in which a target protein is produced is maintained for a longer period than the batch culture method or the fed-batch culture. However, in order to maintain a long-term perfusion culture, particularly a perfusion culture at a high cell density, preparation, use, storage, and disposal of a medium are required. In the perfusion culture, many nutrients are required, and the production cost of the target protein tends to be higher than that in the batch culture and the fed-batch culture. In addition, since it is possible to continue culture while harvesting antibodies out of the system by selecting a membrane pore diameter, it is possible to keep the quality of the antibody high by shortening a retention time for the antibody and reducing chemical change of the antibody, in a culture solution.

It is also possible to perform culture in which the fed-batch culture and the perfusion culture are combined to each other. As an example, it is possible to use fed-batch culture with a bolus feed to maintain the culture of cells in the proliferation period, and subsequently, it is possible to use perfusion culture to produce a target protein.

Perfusion may be in any form of continuous perfusion, phased perfusion, intermittent perfusion, or a combination thereof. Animal cells are retained in the culture product and the used medium that is removed may substantially do not include cells or may have much fewer cells than the culture product. The target protein expressed by a cell culture can be retained in the culture product or harvested by selecting the membrane pore diameter. To prevent the cell density during the culture from becoming excessive, a part of the culture solution may be extracted together with the cells, and the cell density may be reduced (cell bleeding) by adding the same amount of the fresh medium.

In the perfusion culture, the seeded cell density of the cell culture is generally $0.2 \times 10^6$ cells/mL to $3 \times 10^7$ cells/mL, preferably $0.2 \times 10^6$ cells/mL to $1 \times 10^7$ cells/mL, and more preferably $0.5 \times 10^6$ cells/mL to $1 \times 10^7$ cells/mL.

In the perfusion culture, the viable cell rate during the culture period is preferably 80% or more, more preferably 85% or more, and further more preferably 90% or more in the entire period.

In the perfusion culture, the highest attainable cell density is preferably $2 \times 10^8$ cells/mL or less, more preferably $1.5 \times 10^8$ cells/ mL or less, and further more preferably $1.0 \times 10^8$ cells/mL or less.

The perfusion ratio in the perfusion culture is preferably 0.3 vvd to 5.0 vvd, and more preferably 0.3 vvd to 1.5 vvd. Here, vvd represents the following.

$$\text{vvd} = \text{(volume of fresh medium/working volume of reactor/day, feed amount of culture solution/amount of culture solution per day)}$$

The seeded cell density in the cell culture and the highest attainable cell density in the culture can be obtained by measuring the number of cells by a conventional method and dividing the number of cells by the amount of the culture solution.

The viable cell rate (survival rate) during the culture period is obtained by dividing the number of viable cells by (the number of viable cells + the number of dead cells). For example, the number of cells can be measured using Vi-CELL XR (Beckman Coulter, Inc.).

The target protein produced by the above-described culture can be purified. The separation and purification of the target protein may be performed by using separation and purification methods used for normal proteins. For example, it is possible to perform separation and purification of the target protein by appropriately selecting and combining a chromatography column such as affinity chromatography, a filter, ultrafiltration, salting out, dialysis, sodium dodecyl sulfate (SDS) polyacrylamide gel electrophoresis, isoelectric focusing, or the like, but is not limited thereto. The concentration of the target protein obtained as described above can be measured by absorbance measurement or enzyme-linked immunosorbent assay (ELISA) or the like.

Columns used for affinity chromatography include a protein A column, a protein G column, and the like. Examples of the chromatography other than affinity chromatography include ion exchange chromatography, hydrophobic chromatography, gel filtration, reverse phase chromatography, adsorption chromatography, and the like. The chromatography can be performed using liquid phase chromatography such as high performance liquid chromatography (HPLC) or fast protein liquid chromatography (FPLC).

In addition, it is possible to modify the target protein or partially remove the peptide by causing appropriate polypeptide modifying enzyme to act on before or after purification. As the polypeptide modifying enzyme, trypsin, chymotrypsin, lysyl endopeptidase, protein kinase, glucosidase, and the like are used, for example.

[Use of target protein]

In a case where the target protein produced by the method of an aspect of the present invention has useful biological activity as a pharmaceutical, it is possible to produce pharmaceuticals by mixing the target protein with a pharmaceutically acceptable carrier or additive and formulating thereof.

Examples of pharmaceutically acceptable carriers and additives include water, pharmaceutically acceptable organic solvents, collagen, polyvinyl alcohol, polyvinyl pyrrolidone, carboxy vinyl polymer, sodium carboxymethylcellulose, sodium polyacrylate, sodium alginate, aqueous dextran, sodium carboxymethyl starch, pectin, methylcellulose, ethylcellulose, xanthan gum, gum arabic, casein, agar, polyethylene glycol, diglycerin, glycerin, propylene glycol, petrolatum, paraffin, stearyl alcohol, stearic acid, human serum albumin (HSA), mannitol, sorbitol, lactose, pharmaceutically acceptable surfactants, and the like.

For example, in a case of being used as an injectable preparation, it is possible to dissolve the purified target protein in a solvent such as physiological saline, a buffer solution, a glucose solution, or the like, and to use those obtained by adding an anti-adsorption agent such as Tween80, Tween20, gelatin, human serum albumin, or the like thereto. Alternatively, the target protein may be a freeze-dried product used for dissolving and reconstituting the product before use, and sugar alcohol or sugars such as mannitol or glucose can be used as an excipient for freeze-drying, for example.

A method of administering the target protein may be either of oral administration or parenteral administration, but is preferably parenteral administration. Examples thereof include injection (for example, systemic or local administration by intravenous injection, intramuscular injection, intraperitoneal injection, subcutaneous injection, and the like), nasal administration, pulmonary administration, transdermal administration, or the like.

A dose of the target protein is appropriately selected depending on the kind of the target protein, the kind of the disease to be treated or prevented, the age of the patient, the severity of the disease, and the like. Generally, the dose is in a range of 0.001 mg to 1,000 mg per kg of body weight at a time, but is not particularly limited thereto.

The present invention will be described more specifically with reference to the following examples, but the present invention is not limited to the examples.

Examples

<Example 1> Preparation of animal cell

A vector encoding a human SNAT2 gene (OriGene Technologies, Inc., Cat # RC201892) was purchased, and the SNAT2 gene expression cassette (promoter, open reading frame, terminator) was amplified by polymerase chain reaction (PCR) using primer 1 (SEQ ID NO: 3) and primer 2 (SEQ ID NO: 4).

The amplified SNAT2 expression cassette was inserted into a vector co-expressing L chain and H chain of each antibody of antibody 1 (denosumab, https: //www.drugbank.ca/drugs/DB06643), antibody 2 (blosozumab, https://www.drugbank.ca/drugs/DB12560), or antibody 3 (anti-MUC1 antibody, JP 2016-517691A). Construction of the L chain and H chain co-expression vector and introduction thereof into the cell were performed according to Example 2 of JP2016-517691A.

All host CHO-DG44 cells prior to the introduction of genes of the antibody and SNAT2 were cultured in an incubator at 37°C in a 5% $CO_2$ atmosphere. The vector was introduced into host cells $5 \times 10^6$ cells using an electroporation method (Lonza Group AG, 4D-Nucleofector), suspended in 20 mL of an OptiCHO (Life Technologies, 12681011) medium containing 100 times-diluted HT Supplement (100x) (Life Technologies, 11067-030), and then seeded in a T75 flask. After one day, the medium was replaced with an OptiCHO (Life technologies, 12681011) medium containing 175 nmol/L methotrexate (MTX) (Wako Pure Chemical Corporation) without HT supplements. Microscopic observation of the T75 flask was started 3 weeks after the gene introduction, the culture volume was sequentially expanded to 20 mL from the flask in which cell proliferation was observed, seeded in a 125 mL shaking flask (Corning), and cultured with shaking at 140 rpm.

By the above method, antibody 1-SNAT2 cells, antibody 2-SNAT2 cells, and antibody 3-SNAT2 cells that forcibly express the human SNAT2 gene, and the antibody 1, antibody 2, or antibody 3 were established. In addition, gene introduction was carried out in the same manner using a vector expressing antibody L chain and H chain to construct antibody 1 cell, antibody 2 cell, and antibody 3 cell in the control group.

Primer-1: CCGCGGTCATAGCTGTTTCCTGAAC (SEQ ID NO: 3)
Primer-2: CAGCTATGACCGCGGTTAATGGCCACCTCCAGGTGCATTGTGT (SEQ ID NO: 4)

<Example 2> Culture test

Feeding culture test was carried out using 8 levels of antibody 1-SNAT2 cells, antibody 2-SNAT2 cells, antibody 3-SNAT2 cells and antibody 1 cell, antibody 2 cells, and antibody 3 cells.

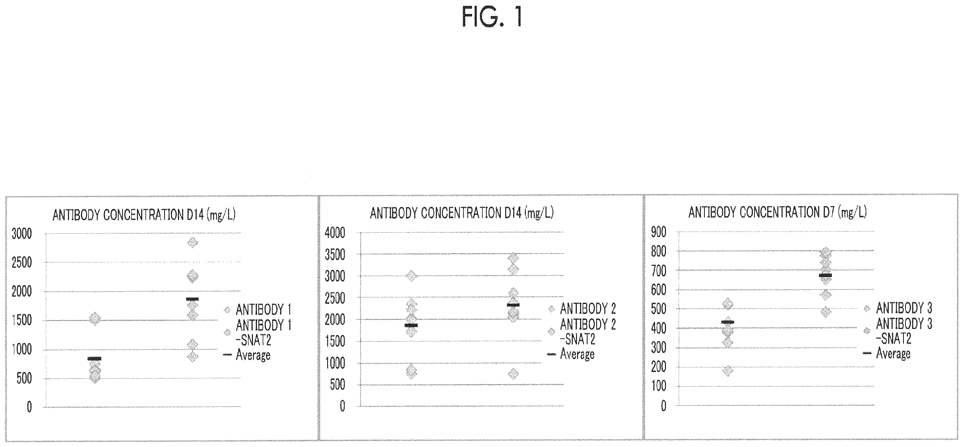

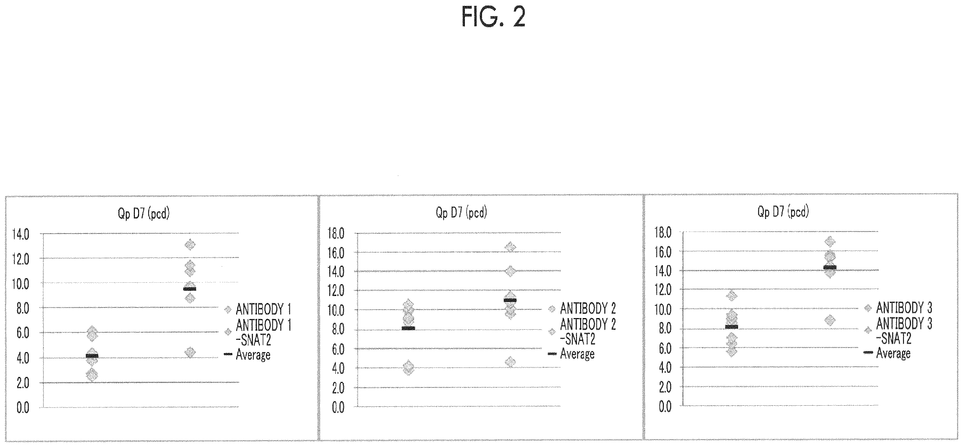

The cells were suspended in 15 mL of an OptiCHO (Life Technologies, 12681011) medium at a cell density of $5 \times 10^5$ cells/mL and automatically cultured with an AMBR15 culture device (Sartorius AG). From the second day to the 13th day after the start of culture, 2% of a feed medium (Cellboost 7a, 7b, GE healthcare) at initial culture volume ratio was added every day. Sampling was performed every third to fourth days to measure the cell density, culture solution components, and an antibody concentration over time. On the 14th day from the start of the culture, the culture solution was harvested, and cells and cell debris were removed using a 0.22 $\mu$m-depth filter (Merck Millipore Corporation). In a case where the antibody concentration in the supernatant and the antibody productivity (Qp) per cell were measured by liquid chromatography, the antibody concentration increased by 121% for antibody 1,25% for antibody 2, and 56% for antibody 3 (Fig. 1 and Table 1). In addition, Qp increased by 127% for antibody 1, 34% for antibody 2, and 74% for antibody 3 (Fig. 2 and

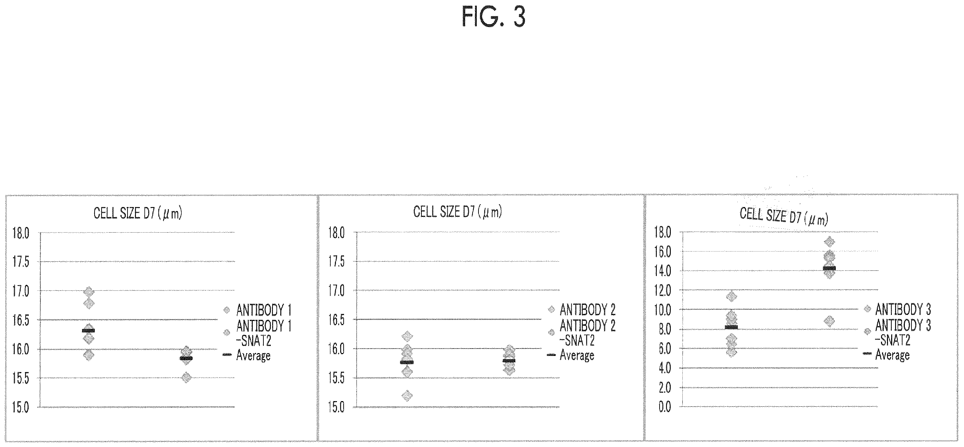

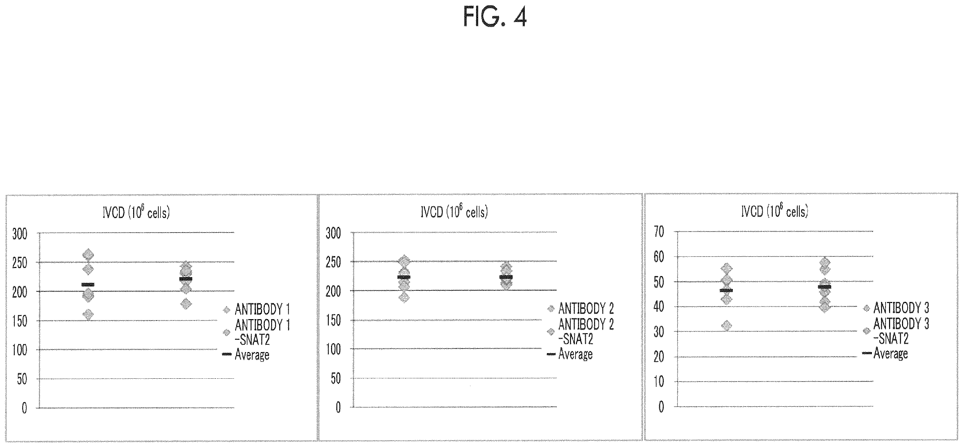

Table 2). It is considered that the antibody productivity was improved since the protein biosynthesis was activated by the expression of SNAT2. On the other hand, as a result of measuring the cell diameter with Vi-CELL (Beckman Coulter, Inc.), surprisingly, it was found that the size of each cell was antibody 1-SNAT2 cells (average 15.8 $\mu$m), antibody 2-SNAT2 cells (average 16.3 $\mu$m), and antibody 3-SNAT2 cells (average 16.5 $\mu$m), and compared to antibody 1 cells (average 16.3 $\mu$m), antibody 2 cells (average 15.8 $\mu$m), and antibody 3 cells (average 16.2 $\mu$m) of the control group, the cell diameter did not necessarily increase and showed almost the same level (Fig. 3 and Table 3). In addition, the cumulative number of viable cells (IVCD) during the culture period showing cell proliferation property was similar to or slightly higher than that of the control group (Fig. 4 and Table 4). In Figs. 1 to 4, only the antibody is shown on the left, and antibody-SNAT2 is shown on the right. This proved that the expression of SNAT2 did not enhance the differentiation of CHO cells, and the cells maintained the cell proliferation ability required for the establishment of antibody-producing cells and the production of antibodies.

The integral viable cell density (IVCD) was measured by the following method. The viable cell density (VCD) is measured by Vi-CELL XR (Beckman Coulter, Inc.) on days 0, 3, 7, 10 and 14, and the respective values are denoted as V0, V3, V7, V10, and V14. The integrated viable cell density (IVCD) is calculated by the following formula.

$$\text{Integral viable cell density (IVCD)} = \{(V0 + V3) \times (3\text{-}0)/2\} + \{(V3 + V7) \times (7\text{-}3)/2\}$$
$$+ \{(V10 + V7) \times (10\text{-}7)/2\} + \{(V14 + V10) \times (14\text{-}10)/2\}$$

[Table 1]

| Antibody concentration D14 comparison | |
|---|---|
| Antibody 1 | 100% |
| Antibody 1-SNAT2 | 221% |
| Antibody concentration D14 comparison | |
| Antibody 2 | 100% |
| Antibody 2-SNAT2 | 125% |
| Antibody concentration D7 comparison | |
| Antibody 3 | 100% |

(continued)

| Antibody concentration D7 comparison | |
|---|---|
| Antibody 3-SNAT2 | 156% |

[Table 2]

| Qp D7 (pcd) comparison | |
|---|---|
| Antibody 1 | 100% |
| Antibody 1-SNAT2 | 227% |
| Qp D7 (pcd) comparison | |
| Antibody 2 | 100% |
| Antibody 2-SNAT2 | 134% |
| Qp D7 (pcd) comparison | |
| Antibody 3 | 100% |
| Antibody 3-SNAT2 | 174% |

[Table 3]

| Cell size D7 comparison | |
|---|---|
| Antibody 1 | 100% |
| Antibody 1-SNAT2 | 97% |
| Cell size D7 comparison | |
| Antibody 2 | 100% |
| Antibody 2-SNAT2 | 100% |
| Cell size D7 comparison | |
| Antibody 3 | 100% |
| Antibody 3-SNAT2 | 102% |

[Table 4]

| IVCD D14 comparison | |
|---|---|
| Antibody 1 | 100% |
| Antibody 1-SNAT2 | 105% |
| IVCD D14 comparison | |
| Antibody 2 | 100% |
| Antibody 2-SNAT2 | 100% |
| IVCD D7 comparison | |
| Antibody 3 | 100% |
| Antibody 3-SNAT2 | 103% |

The antibody productivity Qp [pg/cell/day] per cell was obtained by the following formula.

$$Q_p = \frac{P_{t2} - P_{t1}}{\int_{t1}^{t2} Xdt}$$

Pti [g/L] = concentration of purified product on culture day ti
Xti [cell/day] = viable cell density on culture day ti

An approximate value of integration was calculated by obtaining the area under the proliferation curve from time t1 to t2 as the area of a trapezoid.

$$\int_{t1}^{t2} Xdt \cong \sum \frac{(t_2 - t_1)(X_{t2} + X_{t1})}{2}$$

[Sequence list] The international patent application based on the International Patent Cooperation Treaty.

**Claims**

1. An animal cell comprising: a gene encoding a target protein; and
   a foreign gene encoding SNAT2 and linked to a promoter, wherein the SNAT2 is overexpressed.

2. The animal cell according to claim 1,
   wherein the overexpression is constant.

3. The animal cell according to claim 1 or 2,
   wherein the foreign gene encoding SNAT2 has a base sequence having 90% or more sequence identity with a base
   sequence of SEQ ID NO: 1.

4. The animal cell according to any one of claims 1 to 3,
   wherein the foreign gene encoding SNAT2 has a base sequence of SEQ ID NO: 1.

5. The animal cell according to any one of claims 1 to 4,
   wherein the animal cell is a CHO cell.

6. A method for producing the animal cell according to any one of claims 1 to 5, the method comprising:
   a step of introducing a gene encoding a target protein and a foreign gene encoding SNAT2 and linked to a promoter,
   into animal cells.

7. The method according to claim 6,
   wherein the step of introducing a foreign gene encoding SNAT2 and linked to a promoter is performed by electroporation.

8. A method for producing a target protein, comprising:
   culturing the animal cell according to any one of claims 1 to 5.

9. The method according to claim 8,
   wherein the culture is fed-batch culture.

10. The method according to claim 9,
    wherein a seeded cell density of the cell culture is $0.2 \times 10^6$ cells/mL to $5 \times 10^6$ cells/mL.

11. The method according to claim 10,
    wherein a viable cell rate during a culture period is 60% or more in the entire period.

12. The method according to claim 8,
    wherein the culture is perfusion culture.

13. The method according to claim 12,
    wherein a seeded cell density of the cell culture is $0.2 \times 10^6$ cells/mL to $1 \times 10^7$ cells/mL.

14. The method according to claim 13,
    wherein a viable cell rate during a culture period is 90% or more in the entire period.

FIG. 1

FIG. 2

FIG. 3

FIG. 4

## INTERNATIONAL SEARCH REPORT

| International application No. |
|---|
| PCT/JP2019/048216 |

**A. CLASSIFICATION OF SUBJECT MATTER**
Int. Cl. G12P21/08(2006.01)n, C12N5/10(2006.01)i, G12N15/85(2006.01)i
FI: C12N15/85 Z, C12N5/10 ZNA, C12P21/08

According to International Patent Classification (IPC) or to both national classification and IPC

**B. FIELDS SEARCHED**

Minimum documentation searched (classification system followed by classification symbols)
Int. Cl. C12P21/08, C12N5/10, C12N15/85

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS
(STN); GenBank/EMBL/DDBJ/, GeneSeq; PubMed; Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| X | HATANAKA, T. et al., Amino acid transporter ATA2 | 1-6, 8 |
| Y | is stored at the trans-Golgi network and released | 1-14 |
|  | by insulin stimulus in adipocytes., The Journal of |  |
|  | Biological Chemistry, 2006, vol. 281, no. 51, pp. |  |
|  | 39273-39284, abstract, fig. 2-6, p. 39274, left |  |
|  | column, paragraph [0004] to p. 39274, left column, |  |
|  | paragraph [0002], abstract, fig. 2-6, p. 39274, |  |
|  | left column, paragraph [0004] to p. 39274, left |  |
|  | column, paragraph [0002] |  |
|  |  |  |
| Y | JP 2018-527009 A (PFIZER INC.) 20 September 2018, | 1-14 |
|  | claims 1, 28, 63-65, 69, examples 4, 5, 16 |  |

☒ Further documents are listed in the continuation of Box C.      ☒ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 29.01.2020 | 10.02.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/048216 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | CLONTECH Laboratories Inc., pECFP-C2 Vector Information, 1997, http://www.yrgene.com/documents/vector/pegfp-c2mapmcs.pdf entire text, all drawings | 1-14 |
| A | JP 2018-509904 A (SELEXIS S. A.) 12 April 2018, entire text, all drawings | 1-14 |
| A | WO 2009/054433 A1 (CHUGAI PHARMACEUTICAL CO., LTD.) 30 April 2009, entire text, all drawings | 1-14 |
| A | JP 2006-230252 A (NARA INSTITUTE OF SCIENCE & TECHNOLOGY) 27 September 2006, entire text, all drawings | 1-14 |

Form PCT/ISA/210 (continuation of second sheet) (January 2015)

## INTERNATIONAL SEARCH REPORT
Information on patent family members

| International application No. |
|---|
| PCT/JP2019/048216 |

| Patent Documents referred to in the Report | Publication Date | Patent Family | Publication Date |
|---|---|---|---|
| JP 2018-527009 A | 20.09.2018 | WO 2017/051347 A2 claims 1, 28, 63-65, 69, examples 4, 5, 16 EP 3353313 A1 US 2018/0265904 A1 | |
| JP 2018-509904 A | 12.04.2018 | WO 2016/156574 A1 entire text, all drawings US 2018/0066268 A1 EP 3277818 A1 | |
| WO 2009/054433 A1 | 30.04.2009 | US 2011/0014654 A1 entire text, all drawings EP 2213746 A1 | |
| JP 2006-230252 A | 27.09.2006 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009020144 A

- JP 2016517691 A

**Non-patent literature cited in the description**

- *Nature Communications,* 2015, vol. 6
- *Genes Dev,* 15 June 2002, vol. 16 (12), 1472-1487
- *Cytotechnology,* 2000, vol. 34, 59-70
- *J. Mol. Biol.,* 1990, vol. 215, 403-410

- **SAMBROOK et al.** Molecular Cloning: A Laboratory Manual. Cold Spring Harbor Laboratory Press, 2001, vol. 1, 42-7
- *Nucleic Acids. Res.,* 1990, vol. 18 (17), 5322